(19) Europäisches Patentamt
European Patent Office
Office européen des brevets

(11) Numéro de publication: 0 499 544 B1

(12) # FASCICULE DE BREVET EUROPEEN

(45) Date de publication de fascicule du brevet: **26.04.95**

(51) Int. Cl.⁶: **A61K 31/44**

(21) Numéro de dépôt: **92400389.0**

(22) Date de dépôt: **13.02.92**

(54) **Utilisation de dérivés de tétrahydrothiénopyridine comme inhibiteurs de l'angiogénèse.**

(30) Priorité: **14.02.91 FR 9101783**

(43) Date de publication de la demande:
**19.08.92 Bulletin 92/34**

(45) Mention de la délivrance du brevet:
**26.04.95 Bulletin 95/17**

(84) Etats contractants désignés:
**AT BE CH DE DK FR GB IT LI LU NL PT SE**

(56) Documents cités:
**EP-A- 0 099 802**
**EP-A- 0 281 459**

**CIBA FOUND. SYMP. 100, PAGES 120-131, 1983, PITMAN BOOKS, LONDON, GB R.A. FRASER ET AL. 'Role of mast cells in experimental tumour angiogenesis.'**

**J INVEST DERMATOL, VOL. 81, NO. 6, PAGES 485-488, 1983, R.L. BARNHILL ET AL. 'Biochemical modulation of angiogenesis in the chorioallantoic membrane of the chick embryo.'**

(73) Titulaire: **SANOFI**
**32-34, rue Marbeuf**
**F-75008 Paris (FR)**

(72) Inventeur: **Cazenave, Jean-Pierre**
**31, rue des Fleurs**
**F-67450 Lampertheim (FR)**
Inventeur: **Herbert, Jean-Marc**
**4, rue du Cantou Caout**
**F-31830 Plaisance du Touch (FR)**

(74) Mandataire: **Polus, Camille et al**
**c/o Cabinet Lavoix**
**2, Place d'Estienne d'Orves**
**F-75441 Paris Cedex 09 (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

THROMB RES, VOL. 48, NO. 4, PAGES 403-415, 1987, R. FELISTE ET AL. 'Broad spectrum anti-platelet activity of ticlopidine and PCR 4099 involves the suppression of the effects of released ADP.'

SANG THROMB. VAISS., VOL. 2, NO. 4, PAGES 175-177, 1990, FR J.P. MAFFRAND ET AL. 'NOUVELLES THERAPEUTIOUES ANTIAGREGANTES'

Science, Vol. 235, pages 442-447, 23-1-1987

Science, Vol. 212, pages 1374-1375, 19-6-1981

Biochemical Pharmacology, vol. 44, No. 3, pages 527-532, 1992

## Description

La présente invention concerne l'utilisation de dérivés de tétrahydrothiénopyridine pour la préparation de compositions pharmaceutiques pour prévenir ou traiter les pathologies impliquant ou dépendant d'une néovascularisation et notamment des rétinopathies, l'arthrite rhumatoïde, le psoriasis, certaines tumeurs cancéreuses, les angiomes, le syndrome de Kaposi et les complications du SIDA, les chéloïdes cutanées, les complications des brûlures, les rejets de greffe et l'endométriose.

Ces composés sont, en effet, des inhibiteurs de l'angiogénèse.

Les composés utilisables selon l'invention appartiennent au groupe des composés de formule :

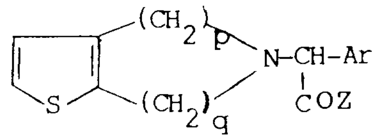

I

dans laquelle

p vaut 2 et q vaut 1 ou p vaut 1 et q vaut 2 ;

Ar représente un phényle non substitué, ou portant un ou plusieurs substituants choisis parmi halogéno, alkyle en $C_1$ à $C_3$, alkoxy en $C_1$ à $C_3$, nitro ou trifluorométhyle; et

Z représente $NR_1R_2$ dans lequel $R_1$ et $R_2$, identiques ou différents, représentent H, alkyle en $C_1$ à $C_4$ éventuellement substitué par $NR_3R_4$, pyridyle ou par phényle substitué ou non par halogéno, alkyle en $C_1$ à $C_3$, alkoxy en $C_1$ à $C_3$, nitro ou trifluorométhyle, ou forment avec l'atome d'azote un hétérocycle saturé, ou Z représente OR dans lequel R est H ou alkyle en $C_1$ à $C_6$ éventuellement substitué par OH ou $NR'_3R'_4$ et $R_3$, $R'_3$, $R_4$ et $R'_4$, représentent chacun H, alkyle en $C_1$ à $C_4$ ou forment avec l'atome d'azote un hétérocycle saturé,

et leurs sels d'addition avec des acides minéraux ou organiques pharmaceutiquement acceptables.

Par hétérocycle saturé on entend piperidino, pyrrolidinyle, morpholino et piperazinyle-1 et, par alkyle et alkoxy, les radicaux à chaîne droite ou ramifié.

Plus précisément les composés utilisables selon l'invention sont choisis parmi l'isomère lévogyre du composé de formule

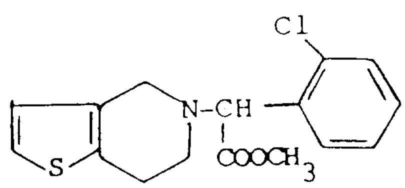

et ses sels d'addition minéraux ou organiques pharmaceutiquement acceptables.

Ce composé a été décrit et trouvé inactif dans EP-A-0 281 459.

Les composés de formule I, dont un certain nombre sont connus et notamment décrits dans EP-A-99 802 et EP-A-281 459, peuvent être préparés de façon classique par action d'un dérivé phénylacétique de formule

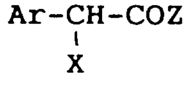

II

dans laquelle X représent Cl ou Br sur la 4,5,6,7-tétrahydrothiéno[3,2-c]pyridine, connue, de formule

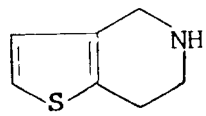

III

ou son isomère, connu, de formule IV

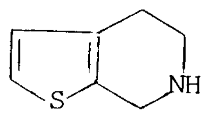

dans un solvant polaire protique, tel qu'un alcool, ou aprotique, tel que l'acétone ou le diméthylformamide, en présence d'une base minérale ou organique pour neutraliser l'acide formé.

Dans le cas où Z est OH, on effectue généralement la substitution de l'azote hétérocyclique avec un composé de formule II dans lequel Z représente un alkoxy et l'ester obtenu sera hydrolysé de façon classique en milieu aqueux de préférence en présence d'une base; on peut aussi préparer à partir des esters d'alkyle les composés dans lesquels Z représente $NR_1R_2$ ou des esters d'alkyle substitués, par des méthodes connues en elles-mêmes.

Les énantiomères des composés de formule I peuvent être obtenus de façon classique, soit à partir du mélange racémique par recristallisations d'un sel avec un acide optiquement actif, tel que l'un des énantiomères de l'acide camphosulfonique-10 ou de l'acide tartrique, soit en faisant réagir sur les composés de formule III ou IV, un seul des énantiomères du composé de formule II, dans des conditions non racémisantes.

Selon un mode de réalisation préféré, on administre le composé utilisable selon l'invention sous la forme d'une composition pharmaceutique contenant l'isomère lévogyre de l'alpha-(tétrahydro-4,5,6,7-thiéno [3,2-c]pyridyl-5)(chloro-2 phényl)-acétate de méthyle ou l'un de ses sels organiques ou minéraux pharmaceutiquement acceptables, au côté des excipients usuels compatibles.

Ces compositions pharmaceutiques seront préparées selon les méthodes galéniques habituelles pour être administrables par voie orale, transmuqueuse ou par injection.

Les doses unitaires et journalières dépendront de l'activité intrinsèque du principe actif et seront adaptées au type et à l'intensité de la pathologie à prévenir ou à traiter, comme à l'âge et au poids du patient. Chez l'adulte pour une administration par voie orale, la dose unitaire peut être comprise entre 5 mg et 500 mg.

Le composé utilisable selon l'invention a une configuration absolue déterminée au niveau du carbone asymétrique puisqu'il est lévogyre.

Selon l'invention, on utilisera de préférence cet énantiomère pur ou associé avec moins de 10% de son homologue.

On sait, en outre, des brevets EP-99802 et EP-281459 que certains des composés de formule 1 ont aussi une activité antiagrégante plaquettaire, dont l'intensité est fonction de la configuration de la molécule autour du carbone asymétrique fixé sur l'azote hétérocyclique, et on préfère utiliser pour préparer les compositions pharmaceutiques de l'invention, inhibitrices de l'angiogénèse, les énantiomères des produits ne présentant qu'une faible activité antiagrégante associée.

On décrit dans ce qui suit des essais pharmacologiques qui montrent l'activité inhibitrice de l'angiogénèse d'un composé représentatif de l'invention ainsi que celles d'autres composés de formule I.

- Essai in vitro :

un milieu nutritif (Dulbecco's modified Eagle medium - DMEM), complémenté avec du sérum de veau foetal (concentration 5% - V/V) et contenant de la glutamine (4 mM), de la pénicilline (100 U/ml), du sulfate de streptomycine (100 µg/ml), introduit dans des puits de 1 ml est ensemencé avec des cellules endothéliales capillaires de cortex cérébral de bovin (20 x $10^3$ cellules/ml), isolées et cultivées comme décrit par D. Gospodarowicz et coll dans J. Cell. physiol. 127, p. 121-136 (1986).

Dès l'adhésion cellulaire, on ajoute 1 ng/ml de facteur de croissance fibroblastique basique bovin, recombinant (rb-FGF) commercialisé par Amersham, facteur connu de stimulation de l'angiogénèse in vivo, puis les solutions à étudier.

Une numération cellulaire est effectuée 5 jours après l'addition de 20 µl d'une solution dans le diméthylsulfoxyde des produits à l'étude, de telle sorte que leur concentration finale dans le milieu soit comprise entre $10^{-4}$ et $10^{-8}$ M; les puits témoins reçoivent 20 µl de solvant.

A partir des résultats obtenus pour plusieurs concentrations de chacun des produits à étudier, on calcule de façon classique à quelle concentration ceux-ci inhibent 50% de la prolifération cellulaire.

Les résultats obtenus figurent dans le tableau I.

- Essai in vivo :

H.F. Dvorak et coll. ont montré que des gels de fibrine induisent une réponse angiogène, in vivo, chez le rat.

Leur technique, décrite dans Laboratory Investigation 57 (6) 673-686 (1987) a été utilisée pour mettre en évidence l'activité inhibitrice de l'angiogénèse des composés de l'invention.

On implante, en sous-cutané, chez le rat, des chambres perforées de plexiglas contenant un gel de fibrine, obtenu par polymérisation de fibrinogène de rat en présence de thrombine. La fibrine est envahie de manière séquentielle et ordonnée par différents types de cellules, tels que des leucocytes, des macrophages, des fibroblastes et des cellules endothéliales, aboutissant à la formation d'un tissu de granulation néovascularisé.

On administre aux rats par voie orale les produits à tester, en suspension dans l'alcool absolu et la gomme arabique à 5% (10 ml/kg), à la dose de 5 et 25 mg/kg/jour pour les composés des exemples 3 et 4, pendant 5 jours avant l'implantation et jusqu'au retrait des chambres. Au bout de 14 jours, un bourgeon de granulation est formé et les chambres sont prélevées afin d'observer la hauteur du bourgeon de granulation ancré dans la matrice et la quantité de néovaisseaux apparus.

Dans une autre expérience pour étudier la régression de l'angiogénèse, les chambres sont implantées sur des rats non traités et l'administration journalière des produits à étudier n'est débutée qu'après 14 jours; l'aspect du bourgeon formé est observé après 8 et 14 jours de traitement.

On a constaté dans les deux séries d'expériences que le composé N° 3 est nettement plus actif que son homologue le composé N° 4, lorsqu'ils sont administrés à la dose de 25 mg/kg/jour et le composé N° 3 est encore très actif à la dose de 5 mg/kg/jour.

L'inhibition de la réponse angiogénique a été évaluée par :

(i) la hauteur et le diamètre des bourgeons de granulation;

(ii) le nombre de néovaisseaux par bourgeon et leur localisation,

(iii) la qualité du tissu de granulation.

La hauteur des bourgeons de granulation est inhibiée de 50% après administration du composé de l'exemple 3 et de 30% après administration de celui de l'exemple 4 (25 mg/kg/jour). Le premier reste efficace à 5 mg/kg/jour (50% d'inhibition) contrairement au second (5 mg/kg/jour). Le nombre de néovaisseaux par bourgeon est significativement abaissé de 90% par le premier (25 mg/kg/jour) et de 60% par le second (25 mg/kg/jour), de 50% en présence du premier à 5 mg/kg/jour.

Le tissu de granulation apparait plus lâche et désorganisé après traitement avec le composé de l'exemple 3.

TABLEAU I

| Composé N° | Formule | isomère | F° (sel) | $CI_{50}$ (nM) |
|---|---|---|---|---|
| 1 | 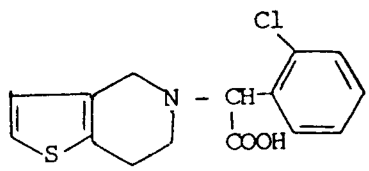 | + | 205°C HCl | 600 |
| 2 | 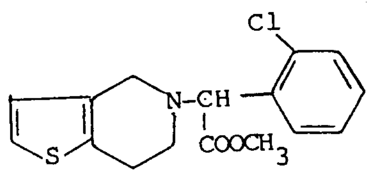 | ± | 140°C HCl | 2,3 |
| 3 | 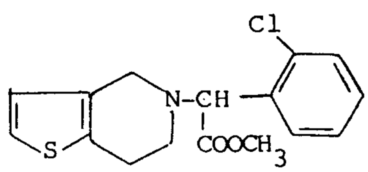 | + | 178°C $H_2SO_4$ | 0,8 |
| 4 | 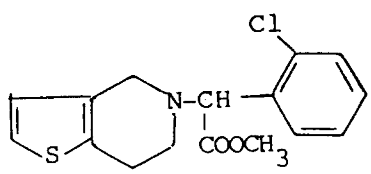 | - | 178°C $H_2SO_4$ | 3,7 |
| 5 | 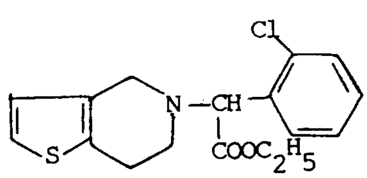 | ± | 180°C HBr | 70 |

TABLEAU I (suite 1)

| Composé N° | Formule | isomère | F° (sel) | $CI_{50}$ (nM) |
|---|---|---|---|---|
| 6 | 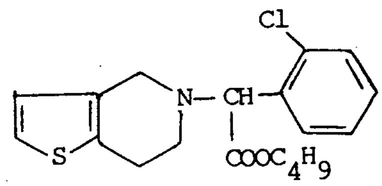 | ± | 155°C $H_2SO_4$ | 220 |
| 7 | 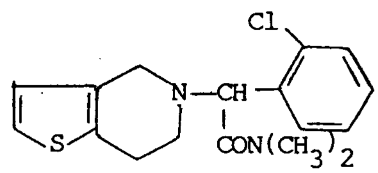 | ± | 95°C base | 80 |
| 8 | 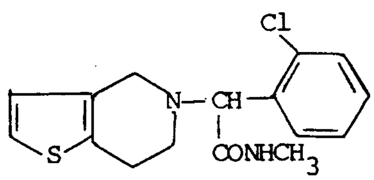 | ± | 137°C base | 50 |
| 9 | 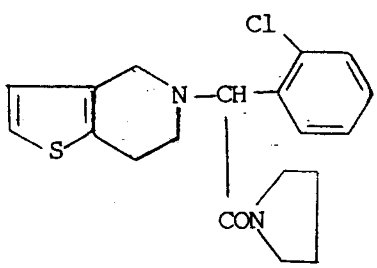 | ± | 130°C base | 320 |
| 10 | 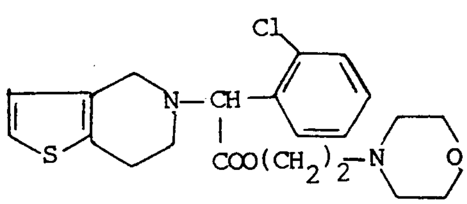 | ± | 203°C $(COOH)_2$ | 20 |

<u>TABLEAU I</u> (suite 2)

| Composé N° | Formule | isomère | F° (sel) | $CI_{50}$ (nM) |
|---|---|---|---|---|
| 11 | 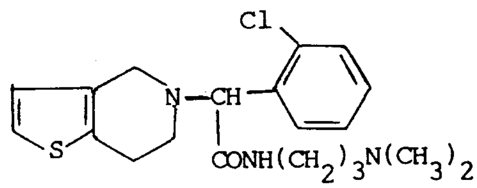 | ± | 210°C 2 HCl | 80 |
| 12 | 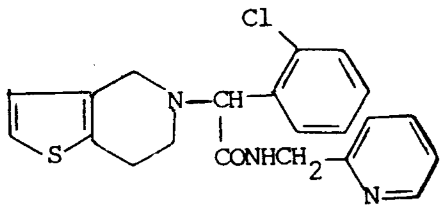 | ± | 122°C base | 30 |
| 13 | 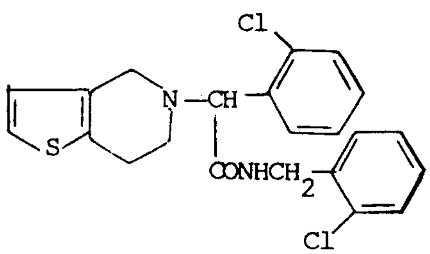 | ± | 116°C base | 1,9 |
| 14 | 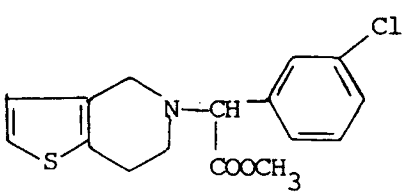 | ± | 186°C HCl | 60 |
| 15 | 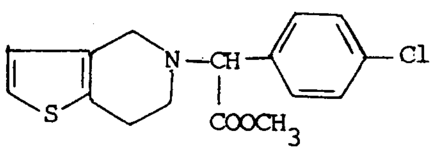 | ± | 202°C HCl | 360 |

TABLEAU I (suite 3)

| Composé N° | Formule | isomère | F° (sel) | $CI_{50}$ (nM) |
|---|---|---|---|---|
| 16 | 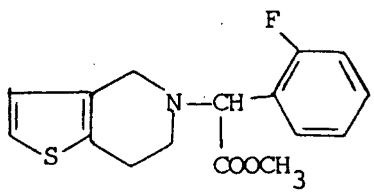 | ± | 100°C HCl | 110 |
| 17 | 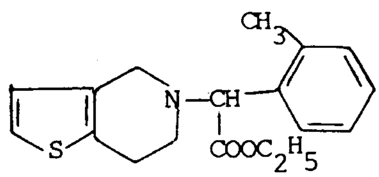 | ± | 188°C $H_2SO_4$ | 110 |
| 18 | 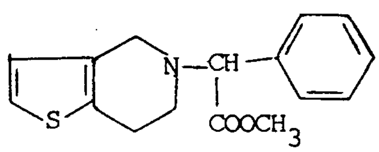 | ± | 200°C HCl | 410 |
| 19 | 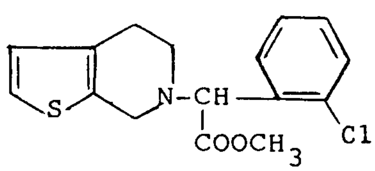 | ± | 150°C HCl | 120 |

## Revendications

**1.** Utilisation de l'isomère lévogyre du composé de formule

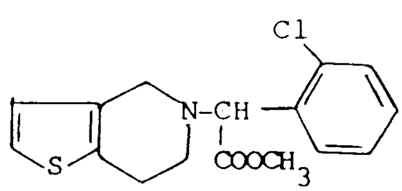

et de ses sels d'addition avec des acides minéraux ou organiques pharmaceutiquement acceptables pour la préparation de compositions pharmaceutiques pour prévenir ou traiter les rétinopathies, l'arthrite rhumatoïde, le psoriasis, certaines tumeurs cancéreuses, les angiomes, le syndrome de Kaposi et les complications du SIDA, les chéloïdes cutanées, les complications des brûlures, les rejets de greffe et l'endométriose.

## Claims

**1.** The use of the laevorotatory isomer of the compound of formula

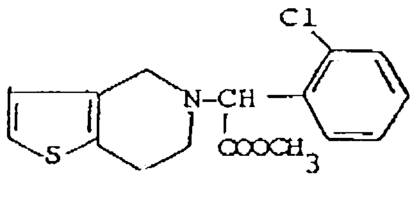

and pharmaceutically acceptable acid addition salts thereof with mineral or organic acids for the preparation of pharmaceutical compositions for the treatment or prevention of retinopathies, rheumatoid arthritis, psoriasis, certain cancerous tumours, angiomas, Kaposi's syndrome and complications of AIDS, cutaneous cheloids, complications of burns, graft rejections and endometriosis.

## Patentansprüche

**1.** Verwendung des linksdrehenden Isomeren der Verbindung der Formel

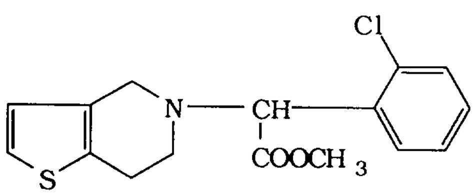

und von dessen Additionssalzen mit anorganischen oder organischen, pharmazeutisch annehmbaren Säuren für die Herstellung von pharmazeutischen Zubereitungen zur Vorbeugung oder Behandlung von Retinopathien, rheumatoider Arthritis, Psoriasis, bestimmten Krebstumoren, Angiomen, des Kaposi-Syndroms und von AIDS-Komplikationen, von Hautkeloiden, von Komplikationen bei Verbrennungen, von Abstoßungen bei Transplantationen und der Endometriose.